Europäisches Patentamt

European Patent Office

Office européen des brevets

(19)

(11) Publication number: **0 060 511**
**B1**

(12) **EUROPEAN PATENT SPECIFICATION**

(45) Date of publication of patent specification: **13.06.84**

(51) Int. Cl.³: **C 07 C 126/02**

(21) Application number: **82101925.4**

(22) Date of filing: **11.03.82**

(54) Process for the preparation of urea.

(30) Priority: **11.03.81 NL 8101174**

(43) Date of publication of application:
**22.09.82 Bulletin 82/38**

(45) Publication of the grant of the patent:
**13.06.84 Bulletin 84/24**

(84) Designated Contracting States:
**AT BE DE FR GB IT NL SE**

(56) References cited:
FR - A - 1 560 985
FR - A - 1 572 656
FR - A - 2 093 882
FR - A - 2 121 068
FR - A - 2 152 018
FR - A - 2 230 626
FR - A - 2 370 037
GB - A - 1 106 024

(73) Proprietor: **UNIE VAN KUNSTMESTFABRIEKEN B.V.**
**Maliebaan 81**
**NL-3581 CG Utrecht (NL)**

(72) Inventor: **Burks, Henk Christiaan**
**Drossaertweide 20**
**NL-6438 HV Oirsbeek (NL)**
Inventor: **Douwes, Adolphe Marie**
**Potterstraat 34**
**NL-6165 AE Geleen (NL)**
Inventor: **Jonckers, Kees**
**Illikhoven 13**
**NL-6121 RH Born (NL)**

(74) Representative: **Roeffen, Wilhelmus Johannes Maria et al,**
**OCTROOIBUREAU DSM Postbus 9**
**NL-6160 MA Geleen (NL)**

Courier Press, Leamington Spa, England.

## Description

The present invention relates to a process for the preparation of urea wherein ammonia and carbon dioxide are reacted at elevated temperature and pressure to form a synthesis reactor effluent containing product urea, ammonium carbamate and excess ammonia, whereafter the ammonium carbamate is decomposed and the resulting ammonia and carbon dioxide together with the excess ammonia are removed from the product urea stream in at least two pressure stages.

In such processes, a major objective has been to devise means for efficiently recovering the ammonia and carbon dioxide released during the ammonium carbamate decomposition, and for recycling it back to the urea synthesis reactor for reaction to a further amount of product urea. One difficulty encountered is that it is generally necessary to expand the urea solution to a pressure substantially below the synthesis reactor pressure in order to remove the final portion of ammonia and carbon dioxide, and to obtain a urea solution which is practically free of ammonium carbamate. The low pressure ammonia and carbon dioxide gas mixture obtained in this reduced pressure decomposition must then be brought back to the synthesis pressure in order to be reutilized, but because of the risk of formation of solid ammonium carbamate, it is very difficult to compress such gas mixtures to such high pressures without circumstantial precautions. Therefore, it has generally been the practice to first condense these gases in the presence of water to form an aqueous ammonium carbamate solution which can then be pumped up to the requisite synthesis pressure and recycled to the reactor as a liquid.

It is also desirable, however, to minimize the water content of this recycled ammonium carbamate solution inasmuch as the introduction of excess water into the urea synthesis reactor decreases the conversion efficiency.

In the process of U.S. Patent No. 3,867,442, the bulk of the ammonium carbamate contained in the urea synthesis reactor is decomposed and stripped out of the urea product stream in a high pressure stage maintained at essentially the same pressure as the urea synthesis reactor, that is, between about 12,000 and 15,000 kPa. It is possible in practice, however to apply higher pressures. In the high pressure stage, the urea reactor effluent is heated to decompose ammonium carbamate and simultaneously stripped with carbon dioxide in a stripping column, and the ammonia and carbon dioxide containing off-gas thus produced is condensed at the same high pressure, and the condensate formed is recycled to the urea reactor. The high pressure stage also includes a washing column operated at about the same pressure wherein ammonia and carbon dioxide contained in an inert gas purge from the top of the reactor are recovered by washing with a dilute solution of ammonium carbamate formed in a low pressure stage, and are returned to the high pressure condensor.

The stripped urea product stream from the bottom of the stripping column is expanded through a pressure reducing valve and led into the rectifying column of a low pressure stage, maintained at a pressure of between about 300 and 400 kPa, wherein a further amount of ammonium carbamate is decomposed and removed from the urea product stream. The resulting low pressure gas mixture of ammonia, carbon dioxide, and water vapor is then condensed, and the aqueous ammonium carbamate solution thus formed is pumped up to the pressure of the high pressure stage and used as the washing liquid in the washing column and ultimately returned to the reactor.

By thus decomposing, condensing, and recycling the bulk of the ammonium carbamate contained in the urea synthesis effluent within the high pressure stage, and decomposing and recovering only a minor portion of the ammonium carbamate at the lower pressure, it has been possible to reduce the amount of water recycled with the carbamate to the reactor as compared to the earlier processes wherein a larger portion of the ammonia and carbon dioxide was recovered at a much lower pressure.

In a further stage of the process the aqueous urea solution, which still contains residual amounts of ammonia and carbon dioxide is usually concentrated by evaporation or crystallization and the resulting gaseous mixture containing the ammonia, carbon dioxide and water is condensed. The ammonia and carbon dioxide are recovered in a desorber and recycled to the low pressure stage.

Applicants have now found that it is possible to make effective and efficient use of the expansion energy released in the transition from the high pressure stage to the low pressure stage, resulting in a reduction in the energy consumption in the process as a whole, and moreover to utilise this expansion energy to yet further reduce the water content of the carbamate returned to the synthesis reactor, thus further enhancing the process efficiency.

Specifically, applicants have found that by interposing a pressure reducing device between the high and low pressure stages in which the energy released by the pressure reduction is converted into kinetic energy, and in turn using this kinetic energy to compress the ammonia and carbon dioxide containing off-gas produced in the low pressure stage to a pressure intermediate that of the high and low pressure stages, a more concentrated ammonium carbamate solution can be formed in the carbamate condenser operating at this higher intermediate pressure. As a result less water is pumped back to the high pressure stage and ultimately into the synthesis reactor.

Preferably, this intermediate pressure will be at least 100 kPa above the pressure in the low pressure stage. At lesser increases in pressure, the advantage obtained is usually to small to be of practical application.

One type of pressure reducing device suitable for use in this invention is an ejector wherein the high pressure liquid from the high pressure stage serves as the motive fluid to aspirate the carbon dioxide and ammonia containing off-gas mixture from a low pressure stage. The resulting gas-liquid mixture is discharged at an intermediate pressure, and the gas phase is separated from the urea product stream, condensed, and pumped to the high pressure stage. The separated urea product stream is fed into the low pressure stage wherein a further amount of ammonium carbamate is decomposed and removed from the solution as the low pressure ammonia and carbon dioxide containing off-gas mixture. It has been found that such an ejector is effective in raising the pressure of this off-gas mixture by generally 150 to 250 kPa, and possibly as much as 1000 kPa, above the pressure in the low pressure stage.

Another type of pressure reducing device found suitable for use in this invention is an expansion turbine which is adapted to drive a compressor for compressing the off-gas mixture from the low pressure stage to the intermediate pressure, at which it is condensed, and the condensate is pumped to the high pressure stage. When using such an expansion turbine, it is possible to increase the off-gas mixture from a pressure of between about 300 and 400 kPa up to an intermediate pressure in the range of between about 800 and 4000 kPa.

In accordance with a first preferred embodiment of the improvement of the present invention, a low pressure off-gas mixture of ammonia and carbon dioxide produced in a desorber is aspirated to the intermediate pressure through an ejector by means of the expanding urea product stream from the high pressure stage. The combined liquid and gas mixture leaving the ejector is introduced into a rectifying column, maintained at the intermediate pressure, wherein an ammonia and carbon dioxide gas mixture is separated from the liquid, and is thereafter condensed at the intermediate pressure to form an ammonium carbamate solution. This ammonium carbamate solution is then pumped to the washing column of the high pressure stage.

The liquid leaving the rectifying column is further reduced in pressure to decompose a further portion of ammonium carbamate which is removed as the ammonia and carbon dioxide containing off-gas mixture and this off-gas mixture is condensed. The resulting condensate is collected in a process condensate tank. The resulting aqueous urea solution is further processed in an evaporation section or a crystallization section. The vapours discharged from this section are condensed and the condensate thus obtained is also collected in the process condensate tank. The process condensate is then treated in a desorber in order to recover the ammonia and carbon dioxide present therein. In this embodiment, it is possible to feed the gas mixture from the desorber, which is generally obtained at a pressure of approximately 300 kPa, to the rectifying column without need for a reflux condenser. Moreover, this embodiment permits the use of low-pressure steam in the desorption column, and makes it possible to carry out the desorption at a pressure lower than 320 kPa, for instance at 100 kPa.

In a second preferred embodiment of the improvement of this invention, the low pressure stage includes a rectifying column and the concentration section includes a desorption column wherefrom gaseous mixtures of ammonia and carbon dioxide are obtained at a pressure of between about 300 and 400 kPa, and compressed in the ejector to an intermediate pressure of between about 450 and 650 kPa by means of the expansion of the urea containing product stream from the high pressure stage. The resulting gas-liquid mixture is fed into a separator wherefrom the gas phase is separated from the liquid and condensed in the carbamate condenser at the intermediate pressure. The separated liquid from the bottom of the separator is further expanded to the pressure of the low pressure stage through an expansion valve and fed into the rectifying column of the low pressure stage wherein further amounts of ammonium carbamate are decomposed and removed from the urea product stream as a low pressure mixture of ammonia and carbon dioxide. The resulting aqueous urea solution is treated in the same way as in the first embodiment. The process condensate obtained is treated in a desorber to recover the ammonia and carbon dioxide.

When carrying out the invention in accordance with this second preferred embodiment, the pressure of the aspirated gases is preferably raised by between about 150 and 250 kPa. At the resulting higher pressure, it is possible to form an ammonium carbamate solution in the carbamate condenser which contains less water than if it were condensed at the pressure of the low pressure stage. Accordingly, less water is fed to the washing column of the high pressure stage, and ultimately less water is recycled to the synthesis reactor which can result in the saving of a considerable amount of steam.

The use of ejectors in urea synthesis processes is not new per se. For instance, in U.S. Patent No. 3,867,442, discussed above, an ejector driven by the high pressure ammonia feed is used within the high pressure stage, to circulate the liquid formed in the bottom of the washing column, together with a small quantity of liquid mixture from the reactor, into the high pressure condenser.

In Japanese Patent Application 41-6334, a urea synthesis solution at 20,000 to 40,000 kPa is expanded through an ejector into a first separator, maintained at a pressure of 1500 to 3500 kPa, while aspirating vapor from a second separator maintained at a low pressure of about 1000 kPa into the first separator, with the objective of minimizing the water content of the solution ultimately recycled to the reactor.

In U.S. Patent No. 3,357,901, a urea synthesis effluent is reduced in carbamate content in a two-part distillation column, the first part operating at a high pressure, such as 1000 to 3000 kPa, the second part operating at a low pressure, such at 0 to 500 kPa. An ejector is used in the line between the synthesis reactor and the high pressure part of the distillation column in order to aspirate the vapor from the low pressure part and to feed it into the high pressure part.

In German Patent Application 1,443,529, the synthesis reaction product from the reactor is degassed two stages, the first degassing taking place at a pressure of 1000 to 5000 kPa, and the second taking place at a pressure of 0 to 800 kPa. The gas mixture obtained in the second degassing stage is compressed to a pressure of 1000 to 5000 kPa, mixed with the gas mixture obtained from the first degassing stage, and the combined gas mixtures are converted into an ammonium carbamate suspension with fresh ammonia which suspension is recycled to the reactor with the aid of a pump. According to one preferred mode of that patent, an ejector placed in the line between the reactor and the first degassing stage is used to aspirate the vapor from the second degassing column and compress it to a pressure of 1000 to 5000 kPa. In accordance with another disclosed mode of operation, the vapor from the second degassing column is aspirated and compressed to a pressure of 1000 to 5000 kPa by means of an ejector installed in the high pressure ammonia line, after which the vapor so compressed can be mixed with the vapor from the first degassing column and fed to the reactor by means of a pump.

In British Patent Specification 509,987, published in 1939, it is suggested that energy can be saved by using an ejector to elevate liquids using the reactor mixture passing out of the autoclave under pressure as the motive fluid, without expending additional energy.

Although the patent literature discussed above suggests that it is possible to make efficient use of the expansion energy within a urea synthesis system by means of an ejector, none of these references suggest the particular processing sequence of the present invention wherein an ejector, or other suitable pressure reducing device as discussed above, is interposed in the urea product stream going from a high pressure ammonium carbamate decomposition stage to a low pressure ammonium carbamate decomposition stage, and the energy thus recovered is utilized to compress vapors released in the low pressure stage to an intermediate pressure whereat they can be condensed to form a carbamate solution of reduced water content for ultimate recycle to the reactor. The net result of the improvement of this invention is an increase in process efficiency, a considerable saving of steam, and a reduced level of capital investment required for process equipment.

The invention will be elucidated in further detail by means of the following embodiments wherein an ejector is used as the pressure reducing device.

Figure 1 schematically shows one possible processing configuration embodying the present invention in which an ejector is utilized to increase the pressure in the carbamate condenser in which the gas mixture from the low pressure stage is condensed to approximately 600 kPa.

Figure 2 schematically illustrates an alternative embodiment wherein the ejector is utilized to aspirate the ammonia and carbon dioxide containing off-gas from the desorption column in the low pressure stage to a rectifying column operating at the intermediate pressure.

In reactor 1, a synthesis solution is formed which contains, in addition to urea and water, an amount of ammonium carbamate and free ammonia. This solution is led from the reactor, via an overflow and line 18, into stripping column 3. In stripping column 3, the synthesis solution is heated indirectly, for instance by means of steam, and is brought into contact and stripped with carbon dioxide supplied through line 13. By means of this stripping treatment, a large portion of the ammonium carbamate initially present in the synthesis solution is decomposed into ammonia and carbon dioxide, and the gases released and dissolved are expelled from the solution. The residual urea product stream, now reduced in ammonium carbamate content, leaves stripping column 3 through line 19. From the top of stripping column 3, a gas mixture containing ammonia, carbon dioxide, the water vapor is removed through line 14, and subsequently partially condensed in condenser 2. The resulting mixture of an aqueous ammonium carbamate solution and free gaseous ammonia and carbon dioxide flows from condenser 2 into the bottom portion of synthesis reactor 1 through line 10. The formation of ammonium carbamate is completed in the reactor, with the optional supply of fresh ammonia through line 7, and a major portion of the ammonium carbamate is therein converted into urea and water.

Inert gaseous components introduced into the process, mainly together with the fresh reaction components and possibly with passivation air, collect in the top of reactor 1 together with equilibrium amounts of gaseous ammonia and carbon dioxide. This gas mixture is purged from the top of the reactor and, in order to recover the valuable components contained therein, it is led through line 11 into washing column 4 wherein it is washed with a dilute ammonium carbamate solution supplied from the low pressure stage through line 29. The ammonia and carbon dioxide present in the mixture from line 11 are nearly all dissolved and condensed in the washing liquid. The non-condensable inert gas is discharged through line 12 via an expansion valve (not illustrated).

Usually washing column 4 is installed at a small height above reactor 1. However, the static pressure produced by the difference in height between the liquid level in the bottom of washing column 4 and the inlet of condenser 2 is insufficient to make the ammonium · carbamate solution flow into the condenser on its own. Because it is desirable that the molar ratio of the ammonia and carbon dioxide in the condenser 2 be maintained at a particular value at a given pressure in order to achieve an optimum condensation temperature, at least a portion of the ammonia required for the synthesis of urea is fed to the condenser through line 8. The energy present in liquid ammonia supplied through lines 6 and 8 is used to make up the pressure deficit between the washing column and the condenser with the aid of jet pump or ejector 5. This jet pump or ejector 5 is also used to aspirate a small amount of the liquid mixture present in the reactor through line 16, and to introduce it as well into condenser 2.

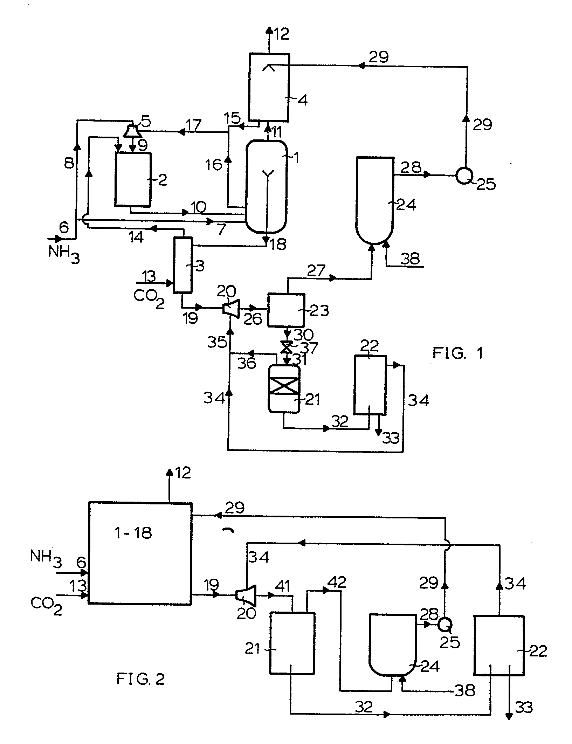

In accordance with the invention, the high pressure solution leaving the bottom of stripper 3 in the high pressure stage, now reduced in ammonium carbamate content, is passed through line 19 and expanded in ejector 20. The energy thereby released is used to aspirate ammonia and carbon dioxide containing off-gases from rectifying column 21 and, optionally, off-gases from the desorption column which forms a part of the concentrating section 22, discharged through lines 36 and 34, respectively, and to ejector 20 through line 35. By means of this ejector, the pressure of the off-gas mixture is raised from the low pressure stage pressure of approximately 300 kPa to an intermediate pressure of between about 400 and 1000 kPa, and fed at this pressure into separator 23. In separator 23, the gas mixture is separated from the liquid, and the liquid is discharged through lines 30 and 31, via expansion valve 37, and is fed into the top of rectifying column 21. The liquid discharged from rectifying column 21 is further degassed, and its urea content raised, in a number of operations, and the gas mixtures thereby separated out are condensed and absorbed to form solutions from which ammonia and carbon dioxide are recovered by processsing apparatus including desorbers. These further operations are known in the art, and are thus represented schematically in Figure 1 by rectangle 22. The urea solution or melt discharged through line 33 may, for instance, be fed to a crystallization section and/or to a prilling or granulation section, which operations are also known in the art and will not be further described here.

The separated gas mixture removed from separator 23 via line 27 is condensed to form a solution of ammonium carbamate in condenser 24, and the solution thus formed leaves condenser 24 through line 28, is increased in pressure by means of carbamate pump 25, and is introduced into washing column 4 of the high pressure stage via line 29. By raising the pressure in carbamate condenser 24 from the approximately 300 kPa of the low pressure stage to a level of 400 to 1000 kPa, the amount of aqueous liquid required to be fed to this condenser via line 38, in order to keep the carbamate in solution, may be decreased since at 300 kPa the solution formed must contain approximately 35 percent water, whereas at 600 kPa, the required water content is only 32 percent, and at 900 kPa is only 27 percent. This decrease in water content of the carbamate formed in condenser 24 is important to the reaction conditions in reactor 1 because the decomposition of ammonium carbamate into urea is an equilibrium reaction in which water is split out, and a low concentration or partial pressure of aqueous vapor in the reactor shifts the equilibrium reaction toward the formation of urea. Thus, when using the improved process, a lesser quantity of liquid need be fed to ammonium carbamate condenser 24 through line 38.

An alternate and simpler embodiment of the improved process of the invention is shown in Figure 2 wherein the expansion of the urea product stream from the high pressure stage is used to aspirate and compress the off-gas mixture from the desorber of process section 22, and feed it into rectifying column 21 at a higher pressure. In practice, the pressure of the off-gas mixture obtained from the desorption column in processing section 22 is generally about 50 kPa lower than the pressure prevailing in rectifying column 21. In order to return the vapor from the desorption column into rectifying column 21 for further recovery and recycling of released ammonia and carbon dioxide, a reflux condenser may be applied. An alternative possibility would be to raise the pressure in the desorption column by heating it with steam of a higher pressure. However, all of these measures require the expenditure of extra energy, and a further difficulty is that often steam of a particular pressure that would be required for the adjustment of the desorption column to the desired pressure is not available. These problems can be overcome by use of an ejector in a configuration such as illustrated in Figure 2.

In Figure 2, the entire high pressure stage including the synthesis reactor is shown as box 1—18 which represents the corresponding components and configuration as shown in Figure 1. The liquid urea product stream leaving the high pressure stage through line 19 is expanded in ejector 20, and aspirates the off-gas mixture from the desorption column of processing section 22 through line 34. The resulting gas liquid mixture, having a pressure intermediate that of the high and low pressure stages, is fed into the rectifying column through line 41. From rectifying column 21, the gas mixture discharged through line 42 is fed to carbamate condenser 24 wherein it is condensed at this intermediate pressure and recycled to the washing column of the high pressure stage by means of lines 28 and 29, and carbamate solution pump 25. The liquid urea product solution discharged from rectifying column 21 through line 32 is further processed in the low pressure system, including the desorption column of processing section 22. The urea product solution discharged from processing section 22 is further processed, for instance in a granulation or prilling section.

The following example illustrates the effect of the improvement of the present invention when incorporated into a urea plant having a configuration as shown in Figure 1, and having an output capacity of approximately 1500 tons per day, wherein the high pressure stage is maintained at urea synthesis reactor pressure of 14,300 kPa. From urea synthesis reactor 1, an effluent was discharged containing 270 tons/day $CO_2$, 200 tons/day $NH_3$, 710 tons/day $H_2O$, and 1532 tons/day urea. This effluent, having a temperature of 170°C and a pressure of 14,300 kPa, was fed to ejector 20 wherein it was expanded to a pressure of 500 kPa resulting in a gas-liquid mixture having a temperature of 120°C. The ejector aspirated an off-gas mixture from the low pressure stage through line 35 so that the gas-liquid mixture discharged through line 26 into separator 23 contained 402 tons/day $CO_2$ 332 tons/day $NH_3$, 900 tons/day $H_2O$, and 1532 tons/day urea. The vapor mixture separated in separator 23, having a temperature of 120°C and a pressure of 500 kPa, and a composition of 293 tons/day $CO_2$, 227 tons/day $NH_3$, and 100 tons/day $H_2O$, was fed to carbamate condenser 24 wherein it was condensed, with the aid of 139 tons/day of $H_2O$ fed into the condenser via line 38, at a pressure of 500 kPa and a temperature of 86°C, to form an aqueous solution of ammonium carbamate. This carbamate solution contained 293 tons/day $CO_2$, 227 tons/day $NH_3$, and 239 tons/day of $H_2O$, and was fed by means of pump 25 through line 29 into the washing column of the high pressure stage.

The urea product stream separated from the gas phase in separator 23 was discharged via expansion valve 37 to a pressure 300 kPa and a temperature of 112°C. This expanded urea product stream, consisting of 1532 tons/day urea, 109 tons/day $CO_2$, 105 tons/day $NH_3$, and 800 tons/day $H_2O$, was fed into rectifying column 21 from which an off-gas mixture of 93 tons/day $CO_2$, 70 tons/day $NH_3$, and 52 tons/day $H_2O$ was discharged through line 36 and aspirated into ejector 20. This off-gas mixture discharged from rectifying column 21 had a pressure of 300 kPa and a temperature of 113°C. This off-gas mixture was mixed with the off-gas obtained from the desorption column in processing section 22 via line 34, which contained 39 tons/day $CO_2$, 53 tons/day $NH_3$ and 138 tons/day of $H_2O$, and had a pressure of 300 kPa and a temperature of 122°C. The resulting combined off-gas mixture, consisting of 132 tons/day $CO_2$, 123 tons/day $NH_3$, and 190 tons/day of $H_2O$, and having a temperature of 117°C and a pressure of 300 kPa was fed to ejector 20 via line 35.

The urea containing product stream discharged from rectifying column 21 through line 32 had a pressure of 300 kPa and a temperature of 135°C, and contained 1532 tons/day urea, 16 tons/day $CO_2$, 35 tons/day $NH_3$, and 748 tons/day $H_2O$. From this urea product stream, further amounts of ammonia and carbon dioxide were recovered by means of the desorption section and recycled to ejector 20.

By operating in accordance with the improvement of this invention as outlined above, the liquid stream recycled to the high pressure stage via line 29 contained only 31.5 percent water, whereas without the use of the ejector this recycled liquid stream contained 34 percent water. Thus, according to this configuration incorporating the improvement of the invention, it is possible to reduce the expenditure of high pressure steam from 848 kg/ton to 829 kg/ton of urea, i.e. by 19 kg/ton of urea, and also to reduce the expenditure of low pressure steam 19 kg/ton urea. This expenditure of low pressure steam is the difference between the amount of low pressure steam produced in the system and the amount of surplus low pressure steam exported from the plant. Additionally, the capital investment for processing equipment could be reduced by approximately one percent when the improvement of this invention was applied.

## Claims

1. Process for the preparation of urea in a synthesis reactor at an elevated temperature and pressure in which process ammonium carbamate is decomposed into ammonia and carbon dioxide and removed from a liquid stream containing product urea in a number of successive pressure stages to produce an aqueous solution of urea product wherein:

in a high pressure stage, ammonium carbamate contained in a first product urea stream is decomposed and the resulting first gaseous mixture containing ammonia and carbon dioxide is separated from a second urea product stream having a reduced ammonium carbamate content;

in further stages, at lower pressures, further amounts of ammonium carbamate are decomposed, further gaseous mixtures containing non-converted ammonia and carbon dioxide are formed, said further gaseous mixtures are condensed and liquid condensates thus formed are pumped to said high pressure stage,

characterized in that the pressure of said second urea product stream is reduced in a pressure reducing device in which the energy released by said pressure reduction is converted into kinetic energy by means of which at least one of said further gaseous mixtures is compressed to a level intermediate the pressure of said high pressure stage and the first of said further stages, whereafter the resulting compressed gaseous mixture is condensed, and the condensate thus formed is pumped to said high pressure stage.

2. Process according to claim 1, characterized in that said high pressure stage includes a stripper, condensor and washing column, and is maintained at substantially the same pressure as the synthesis reactor in the range of between about 12,000 and 15,000 kPa.

3. Process according to claim 1 or 2 characterized in that the first of said further stages includes a rectifying zone and the further gaseous mixture formed in said zone is compressed to said intermediate pressure.

4. Process according to any of the claims 1 to 3, characterized in that said further stages include a concentration stage in which the aqueous solution of urea product is concentrated, the resulting gaseous mixture containing ammonia, carbon dioxide and water removed thereby is condensed, the condensate thus formed is treated in a desorption zone to recover ammonia and carbon dioxide as a further gaseous mixture, and said further gaseous mixture is compressed to said intermediate pressure.

5. Process according to claim 1 or 2 characterized in that said pressure reducing device is an ejector by means of which at least one of said further gaseous mixtures is aspirated into said second urea product stream and the resulting gas-liquid mixture is introduced into a separator maintained at said intermediate pressure wherefrom the separated gas containing ammonia and carbon dioxide is condensed at said intermediate pressure and the condensate thus formed is pumped to said high pressure stage, and the separated liquid containing product urea and ammonium carbamate is fed into said the first of said further stages.

6. Process according to claim 5 characterized in that said further stages include a rectifying zone and a desorption zone, and said separated liquid is expanded from said intermediate pressure to the pressure of the first of said further stages and treated in said rectifying zone to decompose a further amount of ammonium carbamate, the aqueous solution of urea product is concentrated, the resulting gaseous mixture containing ammonia, carbon dioxide and water removed thereby is condensed, the condensate thus formed is treated in said desorption zone to recover a gaseous mixture containing ammonia and carbon dioxide and the resulting gaseous mixtures containing ammonia and carbon dioxide from said rectifying and desorption zones are aspirated by means of said ejector and compressed to said intermediate pressure.

7. Process according to claim 6 characterized in that said intermediate pressure is at least 100 kPa above the pressure in the first of said further stages.

8. Process according to claim 7 characterized in that said intermediate pressure is 150 to 250 kPa above the pressure in the first of said further stages.

9. Process according to any of claims 5 to 8 characterized in that said separator is a rectifying column maintained in said intermediate pressure wherefrom the separated gas containing ammonia and carbon dioxide is condensed in said intermediate pressure, and the condensate thus formed is pumped to said high pressure zone, and said aqueous solution of urea product is concentrated, the resulting gaseous mixture containing ammonia, carbon dioxide and water removed thereby is condensed, the condensate thus formed is treated in a desorption zone to recover ammonia and carbon dioxide as a further gaseous mixture and said further gaseous mixture is aspirated by said ejector and fed into the rectifying column.

10. Process according to any of claims 1 to 4 characterized in that said pressure reducing device is an expansion turbine adapted to drive a compressor by means of which at least one of said further gaseous mixture is compressed to said intermediate pressure.

11. Process of claim 10 characterized in that said low pressure stage is maintained at a pressure of between about 300 and 400 kPa, and said intermediate pressure is in the range of between about 800 and 4000 kPa.

**Patentansprüche**

1. Verfahren zur Herstellung von Harnstoff in einem Synthesereaktor bei erhöhter Temperatur und erhöhtem Druck, bei welchem Verfahren Ammoniumcarbamat zu Ammoniak und Kohlendioxid zersetzt und aus einem Produktharnstoff enthaltenden Flüssigkeitsstrom in einer Anzahl von aufeinanderfolgenden Druckstufen entfernt wird, um eine wässerige Lösung von Harnstoffprodukt zu erzeugen, wobei:

in einer Hochdruckstufe Ammoniumcarbamat, das in einem ersten Produktharnstoffstrom enthalten ist, zersetzt und das resultierende erste, Ammoniak und Kohlendioxid enthaltende gasförmige Gemisch aus einem zweiten Harnstoffproduktstrom mit herabgesetztem Ammoniumcarbamatgehalt abgetrennt wird;

in weiteren Stufen, bei niedrigeren Drücken, weitere Mengen an Ammoniumcarbamat zersetzt, weitere, nicht umgewandeltes Ammoniak und Kohlendioxid enthaltende gasförmige Gemische gebildet, diese weiteren gasförmigen Gemische kondensiert und so gebildete flüssige Kondensate in die Hochdruckstufe gepumpt werden,

dadurch gekennzeichnet, daß man den Druck des zweiten Harnstoffproduktstromes in einer Druckverminderungsvorrichtung herabsetzt, in welcher die durch diese Druckverminderung freigesetzte Energie in kinetische Energie umgewandelt wird, mit deren Hilfe man mindestens eines der genannten gasförmigen Gemische auf einen zwischen dem Druck der Hochdruckstufe und dem Druck der ersten der genannten weiteren Stufen liegenden Druckwert komprimiert, wonach man das resultierende komprimierte Gasgemisch kondensiert und das so gebildete Kondensat in die Hochdruckstufe pumpt.

2. Verfahren nach Anspruch 1, dadurch gekennzeichnet, daß die Hochdruckstufe einen Stripper, einen Kondensator und eine Waschkolonne umfaßt und im wesentlichen auf demselben Druck wie der Synthesereaktor im Bereich von ungefähr 12.000 und 15.000 kPa gehalten wird.

3. Verfahren nach Anspruch 1 oder 2, dadurch gekennzeichnet, daß die erste der weiteren Stufen eine Rektifikationszone umfaßt und daß weitere, in dieser Zone gebildete gasförmige Gemische auf den Zwischendruck komprimiert werden.

4. Verfahren nach einem der Ansprüche 1 bis 3, dadurch gekennzeichnet, daß die weiteren Stufen eine Konzentrierungsstufe umfassen, in welcher man die wässerige Lösung des Harnstoffprodukts konzentriert, das resultierende, dabei entfernte gasförmige Gemisch, das Ammoniak, Kohlendioxid und Wasser enthält, kondensiert, das so gebildete Kondensat in einer Desorptionszone behandelt, um Ammoniak und Kohlendioxid als ein weiteres gasförmiges Gemisch rückzugewinnen, und dieses weitere gasförmige Gemisch auf den Zwischendruck komprimiert.

5. Verfahren nach Anspruch 1 oder 2, dadurch gekennzeichnet, daß die Druckverminderungsvorrichtung ein Ejektor ist, mittels welchem mindestens eines der weiteren gasförmigen Gemische in den zweiten Harnstoffproduktstrom eingesaugt wird, und daß man das resultierende Gas-Flüssigkeitsgemisch in einen auf dem Zwischendruck gehaltenen Separator einbringt, worin das abgetrennte, Ammoniak und Kohlendioxid enthaltende Gas bei dem Zwischendruck kondensiert wird, und man das so gebildete Kondensat in die Hochdruckstufe pumpt und die abgetrennte, Harnstoffprodukt und Ammoniumcarbamat enthaltende Flüssigkeit in die erste der weiteren Stufen einbringt.

6. Verfahren nach Anspruch 5, dadurch gekennzeichnet, daß die weiteren Stufen eine Rektifikationszone und eine Desorptionszone umfassen und daß man die abgetrennte Flüssigkeit von dem Zwischendruck auf den Druck der ersten der weiteren Stufen expandiert und in der Rektifikatonszone behandelt, um eine weitere Menge an Ammoniumcarbamat zu zerzetzen, die wässerige Lösung des Harnstoffprodukts konzentriert, das resultierende, dabei entfernte gasförmige Gemisch, das Ammoniak, Kohlendioxid und Wasser enthält, kondensiert, das so gebildete Kondensat in der Desorptionszone behandelt, um ein Ammoniak und Kohlendioxid enthaltendes gasförmiges Gemisch rückzugewinnen, und die resultierenden Ammoniak und Kohlendioxid enthaltenden gasförmigen Gemische aus der Rektifikations- und Desorptionszone mittels des Ejektors ansaugt und auf den Zwischendruck komprimiert.

7. Verfahren nach Anspruch 6, dadurch gekennzeichnet, daß der Zwischendruck um mindestens 100 kPa höher ist, als der Druck in der ersten der weiteren Stufen.

8. Verfahren nach Anspruch 7, dadurch gekennzeichnet, daß der Zwischendruck um 150 bis 250 kPa höher ist, als der Druck in der ersten der weiteren Stufen.

9. Verfahren nach einem der Ansprüche 5 bis 8, dadurch gekennzeichnet, daß der Separator eine auf dem Zwischendruck gehaltene Rektifikatonskolonne ist und daß man das darin abgetrennte, Ammoniak und Kohlendioxid enthaltende Gas bei diesem Zwischendruck kondensiert und das so gebildete Kondensat in die Hochdruckzone pumpt und die wässerige Lösung des Harnstoffprodukts konzentriert, das resultierende, dabei entfernte, gasförmige Gemisch, das Ammoniak, Kohlendioxid und Wasser enthält, kondensiert und das so gebildete Kondensat in einer Desorptionszone behandelt, um Ammoniak, und Kohlendioxid als ein weiteres gasförmiges Gemisch rückzugewinnen, welches durch den Ejektor angesaugt und in die Rektifikationskolonne eingeführt wird.

10. Verfahren nach einem der Ansprüche 1 bis 4, dadurch gekennzeichnet, daß die Druckverminderungsvorrichtung eine Expansionsturbine ist, die zum Antreiben eines Kompressors eingerichtet ist, mittels welchem mindestens eines der weiteren Gasgemische auf den Zwischendruck komprimiert wird.

11. Verfahren nach Anspruch 10, dadurch gekennzeichnet, daß die Niederdruckstufe auf einem Druck zwischen ungefähr 300 und 400 kPa gehalten wird und der Zwischendruck im Bereich zwischen ungefähr 800 bis 4000 kPa liegt.

## Revendications

1. Procédé pour la préparation de l'urée dans un réacteur de synthèse à une température et une pression élevées dans lequel procédé du carbamate d'ammonium est décomposé en ammoniac et en dioxyde de carbone et éliminé d'un courant liquide contenant l'urée produite dans un certain nombre de stades sous pression successifs pour produire une solution aqueuse d'urée produite, dans lequel:

— dans un stade à pression élevée, le carbamate d'ammonium contenu dans un premier courant d'urée produite est décomposé et le premier mélange gazeux obtenu contenant de l'ammoniac et du dioxyde de carbone est séparé d'un second courant d'urée produite ayant une teneur réduite en carbamate d'ammonium;

— dans des stades ultérieures, à des pressions plus basses, des quantités complémentaires de carbamate d'ammonium sont décomposées, des mélanges gazeux complémentaires contenant de l'ammoniac et du dioxyde de carbone non transformés sont formés, lesdits mélanges gazeux complémenatires sont condensés et les condensats liquides ainsi formés sont pompés dans ledit stade à pression élevée,

caractérisé en ce que la pression dudit second courant d'urée produite est réduite dans un dispositif abaissant la pression dans lequel l'énergie libérée par ledit abaissement de la pression est transformée en énergie cinétique grâce à laquelle au moins un desdits mélanges gazeux complémentaires est comprimé à une valeur intermédiaire entre la pression dudit stade à pression élévée et le premier desdits stades ultérieures, après quoi le mélange gazeux comprimé obtenu est condensé et le condensat ainsi formé est pompé dans ledit stade à pression élevée.

2. Procédé selon la revendication 1, caractérisé en ce que ledit stade à pression élevée comprend une colonne d'entraînement, un condenseur et une colonne de lavage et est maintenu pratiquement à la même pression que le réacteur de synthèse dans la gamme entre environ 12 000 et 15 000 kPa.

3. Procédé selon la revendication 1 ou 2, caractérisé en ce que le premier desdits stades ultérieures comprend une zone de rectification et le mélange gazeux complémentaire formé dans ladite zone est comprimé à ladite pression intermédiaire.

4. Procédé selon l'une quelconque des revendications 1 à 3, caractérisé en ce que lesdits stades ultérieures comprennent un stade de concentration dans lequel la solution aqueuse d'urée produite est concentrée, le mélange gazeux obtenu contenant de l'ammoniac, du dioxyde de carbone et de l'eau ainsi éliminé est condensé, le condensat ainsi formé est traité dans une zone de désorption pour récupérer l'ammoniac et le dioxyde de carbone sous forme d'un mélange gazeux complémentaire et ledit mélange gazeux complémentaire est comprimé à ladite pression intermédiare.

5. Procédé selon la revendication 1 ou 2, caractérisé en ce que ledit dispositif abaissant la pression est un éjecteur au moyen duquel au moins un desdits mélanges gazeux complémentaires est aspiré dans ledit second courant d'urée produite et le mélange gaz-liquide obtenu est introduit dans un séparateur maintenu à ladite pression intermédiaire où le gaz séparé contenant de l'ammoniac et du dioxyde de carbone est condensé à ladite pression intermédiaire et le condensat ainsi formé est pompé dans ledit stade à pression élevée et le liquide séparé contenant l'urée produite et le carbamate d'ammonium est introduit dans le premier desdits stades ultérieurs.

6. Procédé selon la revendication 5, caractérisé en ce que lesdits stades ultérieurs comprennent une zone de rectification et une zone de désorption et ledit liquide séparé est détendu de ladite pression intermédiare à la pression du premier desdits stades ultérieurs et traité dans ladite zone de rectification pour décomposer une quantité complémentaire de carbamate d'ammonium le solution aqueuse d'urée produite est concentrée, le mélange gazeux contenant l'ammoniac, le dioxyde de carbone et l'eau ainsi éliminé est condensé, le condensat ainsi formé est traité dans ladite zone de désorption pour récupérer un mélange gazeux contenant de l'ammoniac et du dioxyde de carbone et les mélanges gazeux obtenus contenant de l'ammoniac et du dioxyde de carbone desdites zones de rectification et de désorption sont aspirés au moyen dudit éjecteur et comprimés à ladite pression intermédiaire.

7. Procédé selon la revendication 6, caractérisé en ce que ladite pression intermédiaire est supérieure d'au moins 100 kPa à la pression dans le premier desdits stades ultérieures.

8. Procédé selon la revendication 7, caractérisé en ce que ladite pression intermédiaire est supérieure de 150 à 250 kPa à la pression dans le premier desdits stades ultérieurs.

9. Procédé selon l'une quelconque des revendications 5 à 8, caractérisé en ce que ledit séparateur est une colonne de rectification maintenue à ladite pression intermédiaire où le gaz séparé contenant de l'ammoniac et du dioxyde de carbone est condensé à ladite pression intermédiaire et le condensat ainsi formé est pompé dans ladite zone à pression élevée et ladite solution aqueuse d'urée produite est concentrée, le mélange gazeux obtenu contenant de l'ammoniac, du dioxyde de carbone et de l'eau ainsi éliminé est condensé, le condensat ainsi formé est traité dans une zone de désorption pour récupérer l'ammoniac et le dioxyde de carbone sous forme d'un mélange gazeux complémentaire et ledit mélange gazeux complémentaire est aspiré par ledit éjecteur et introduit dans la colonne de rectification.

10. Procédé selon l'une quelconque des revendications 1 à 4, caractérisé en ce que ledit dispositif abaissant la pression est une turbine à détente conçue pour entraîner un compresseur au moyen duquel au moins un desdits mélanges gazeux complémentaires est comprimé à ladite pression intermédiaire.

11. Procédé selon la revendication 10, caractérisé en ce que ledit stade à basse pression est maintenu à une pression entre environ 300 et 400 kPa et ladite pression intermédiaire est dans la gamme entre environ 800 et 4 000 kPa.

FIG. 1

FIG. 2